# EUROPEAN PATENT APPLICATION

(11) **EP 4 717 254 A1**
(43) Date of publication of application: **01.04.2026**
(21) Application number: 24811333.4
(22) Date of filing: 14.05.2024
(51) Int. Cl.: A61K 8/46, A61K 8/29, A61Q 17/04

(54) **UV BLOCKING COSMETIC COMPOSITION COMPRISING UV BLOCKING MATERIAL CONSISTING OF ORGANIC-INORGANIC COMPLEX**

(30) Priority: 23.05.2023 KR 20230066108
(71) Applicant: Kolmar Korea Co., Ltd., Sejong 30003 (KR)
(72) Inventor: YOO, Cha Young, Seoul 06800 (KR); LEE, Nam Hee, Seoul 06800 (KR); KIM, Yong Woo, Seoul 06800 (KR)
(74) Representative: Mewburn Ellis LLP
(86) International application number: PCT/KR2024/006558
(87) International publication number: WO 2024/242394

(57) **Abstract**

The present disclosure relates to a cosmetic composition including a UV blocking material consisting of an organic-inorganic complex, an organic thickener, and an inorganic thickener, and more particularly, to a UV blocking cosmetic composition including a novel UV blocking material in the form of an organic-inorganic complex formed from terephthalylidene dicamphor sulfonic acid, which is a water-soluble organic UV blocking agent, and titanium dioxide, which is an inorganic UV blocking agent. The cosmetic composition for UV blocking according to the present disclosure contains a novel UV blocking agent in the form of an organic-inorganic complex, thereby having an excellent UV blocking effect, and has a reduced whitening phenomenon and aggregation phenomenon, and thus can be usefully used as a cosmetic composition for UV blocking.

## Description

### [Technical Field]

The present disclosure relates to a cosmetic composition including a UV blocking material consisting of an organic-inorganic complex, an organic thickener, and an inorganic thickener, and more particularly, to a UV blocking cosmetic composition including a novel UV blocking material in the form of an organic-inorganic complex formed from terephthalylidene dicamphor sulfonic acid, which is a water-soluble organic UV blocking agent, and titanium dioxide, which is an inorganic UV blocking agent.

### [Background Art]

Sunlight has beneficial effects of inducing photosynthesis in plants and vitamin D synthesis in animals, promoting bone growth to inhibit rickets, and promoting differentiation of epidermal cells to strengthen the skin barrier. On the other hand, the sunlight causes sunburn and generates wrinkles by causing erythema, collagen destruction and pigmentation to promote photoaging, and may even have a harmful effect of causing skin cancer.

UV rays are rays with a wavelength range of 200 to 400 nm in sunlight, accounting for about 6% of sunlight reaching the Earth's surface. Depending on the wavelength range, the UV rays are classified into UV-A (320 to 400 nm) with the longest wavelength, UV-B (290 to 320 nm) with a medium wavelength, and UV-C (200 to 290 nm) with the shortest wavelength. The UV-A accounts for 90% or more of the UV rays that reach the Earth's surface. The UV-A has a relatively small effect on living things, but accelerates skin aging when continuously exposed, and the UV-B is mostly absorbed in the ozone layer and rarely reaches the ground, but damages DNA of skin keratinocytes to cause skin cancer when exposed.

The long-wavelength UV-A may penetrate deep into the skin and cause "immediate tanning" within 12 hours of exposure. In addition, the UV-B has a shorter wavelength than UV-A and does not penetrate deep into the skin, but has high energy to cause sunburn. Short-wavelength UV-C is mostly absorbed in the ozone layer, but may cause skin cancer when continuously exposed. Recently, a risk of the UV rays is increasing due to the destruction of the ozone layer caused by environmental pollution.

Currently, a UV blocking agent for blocking UV rays is classified as a functional cosmetic, and various studies thereon have been conducted. The UV blocking agents are classified into a physical blocking agent and a chemical blocking agent. The physical blocking agent scatters UV rays to prevent the UV rays from penetrating into the skin, while the chemical blocking agent absorbs the UV rays to prevent the UV rays from penetrating into the skin.

The UV blocking agents may be classified into an organic UV blocking agent and an inorganic UV blocking agent. The organic UV blocking agent typically includes chemical blocking agents that convert light into heat, and the inorganic UV blocking agent typically includes physical blocking agents that reflect, scatter, and absorb light. Unlike basic cosmetics, the UV blocking agents are primarily used to reduce UV rays in the upper epidermis, i.e., the outermost layer of the skin. However, organic UV blocking agents such as avobenzone have small molecules and thus can penetrate into the skin. The organic UV blocking agents have advantages of less whitening and various absorption wavelengths, but may cause skin problems or also cause side effects such as eye irritation when applied around the eyes in a sensitive case. On the other hand, the inorganic UV blocking agents are relatively advantageous in safety and have good blocking ability, but may cause problems such as a whitening phenomenon due to white pigments with a high refractive index. Due to a recent trend of eco-friendly cosmetic materials, preference is high in Korea for UV blocking products in a formulation of 'physical sunscreen' consisting of only an inorganic UV blocking agent as a functional ingredient.

Titanium dioxide (TiO₂) and zinc oxide (ZnO) are used as the inorganic UV blocking agent, but have several disadvantages. First, the energy band gaps of titanium dioxide and zinc oxide are 3.0 eV and 3.2 eV, respectively, which are advantageous for absorbing UVB and UVA2, so that titanium dioxide and zinc oxide alone cannot absorb UVA1 with a medium wavelength. Second, the refractive indices of titanium dioxide and zinc oxide are 2.7 and 2.2, respectively, and thus when applied to the skin, a whitening phenomenon may be noticeable. Third, titanium dioxide and zinc oxide have a strong photocatalytic ability to degrade or denature organic materials, particularly pigments, under light energy, which may cause ingredient denaturation and pigment deposition in the formulation. In particular, when the photocatalytic ability is high, the surface needs to be covered with a second material due to safety, and in the case of titanium dioxide, the surface is covered with aluminum oxide (Al₂O₃) or silicon dioxide (SiO₂) at up to 20 weight fractions or more. However, when surface treatment is performed as such, the powder texture is heavy and does not spread smoothly, so that the usability is stiff, and the whitening phenomenon cannot be significantly reduced. Therefore, there is a need to develop a UV blocking agent composition capable of compensating for the shortcomings.

Currently, Korean Patent Registration No. 10-2354352 discloses an oil-in-water cosmetic composition including an inorganic UV blocking agent, and Korean Publication Patent No. 10-2013-0047555 discloses an organic-inorganic complex powder for UV blocking and a cosmetic composition containing the same. However, there is disclosed no UV blocking cosmetic composition that includes an organic-inorganic complex UV blocking material of the present disclosure and has an excellent blocking effect and a significantly reduced aggregation phenomenon.

### [Prior Arts]

(Patent Document 1) Korean Patent Registration No. 10-2354352
(Patent Document 2) Korean Patent Publication No. 10-2013-0047555

### [Disclosure]

### [Technical Problem]

An object of the present disclosure is to provide a cosmetic composition including a UV blocking material consisting of an organic-inorganic complex, an organic thickener, and an inorganic thickener.

### [Technical Solution]

Accordingly, the present inventors developed a UV blocking cosmetic composition, which included a novel UV blocking material in the form of an organic-inorganic complex formed from terephthalylidene dicamphor sulfonic acid, which was a water-soluble organic UV blocking agent with an excellent UV blocking effect, and titanium dioxide, which was an inorganic UV blocking agent, found that when the composition was formulated into a cosmetic composition together with an organic thickener and an inorganic thickener, an aggregation phenomenon and a whitening phenomenon were significantly reduce, and then completed the present disclosure.

In order to achieve the object, an aspect of the present disclosure provides a UV blocking cosmetic composition including a UV blocking material consisting of an organic-inorganic complex, an organic thickener, and an inorganic thickener, in which the UV blocking material consisting of the organic-inorganic complex is formed from terephthalylidene dicamphor sulfonic acid and titanium dioxide.

### [Advantageous Effects]

According to the present disclosure, the UV blocking cosmetic composition contains a novel UV blocking agent in the form of an organic-inorganic complex, thereby having an excellent UV blocking effect, and having reduced whitening phenomenon and aggregation phenomenon, and thus can be usefully used as a cosmetic composition for UV blocking.

### [Description of Drawings]

FIG. 1 is a diagram illustrating comparing results of observing a whitening phenomenon with respect to compositions of Example and Comparative Examples according to the present disclosure.
FIG. 2 is a diagram illustrating comparing results of observing temporal changes with respect to compositions of Example and Comparative Examples according to the present disclosure.

### [Best Mode]

Hereinafter, the present disclosure will be described in more detail through embodiments. These embodiments are to explain the present disclosure in more detail, and it will be apparent to those skilled in the art that the scope of the present disclosure is not limited by these embodiments in accordance with the gist of the present disclosure.

The present disclosure provides a cosmetic composition including a UV blocking material consisting of an organic-inorganic complex, an organic thickener, and an inorganic thickener.

The UV blocking material consisting of the organic-inorganic complex may be formed from terephthalylidene dicamphor sulfonic acid and titanium dioxide, and may be contained in an amount of 1 to 10 wt%, preferably 3 wt%, based on the total weight of the composition, but is not limited thereto.

In one embodiment of the present disclosure, the organic thickener may include a first thickener and a second thickener.

The first thickener may be one or a mixture of one or more selected from the group consisting of hydroxypropyl methylcellulose stearoxy ether, hydroxyethyl cellulose, carboxymethyl cellulose, and hydroxyethyl methyl cellulose, and may be contained in an amount of 0.01 to 1 wt% based on the total weight of the composition, but is not limited thereto.

The second thickener may be one or more selected from the group consisting of cellulose gum, xanthan gum, gellan gum, guar gum, dehydroxanthan gum, carob bean gum, tamarind seed gum, mastic gum, carob gum, diutan gum, and tara gum, and preferably xanthan gum, and may be contained in an amount of 0.01 to 2 wt% based on the total weight of the composition, but is not limited thereto.

In one embodiment of the present disclosure, the inorganic thickener may be hectorite or bentonite, and may be contained in an amount of 0.01 to 2 wt% based on the total weight of the composition, but is not limited thereto.

In one embodiment of the present disclosure, the cosmetic composition may consist of a water phase part and an oil phase part, and the water phase part may further include one or more ingredients selected from the group consisting of a solvent, a skin conditioning agent, an emulsion stabilizer, a pH adjusting agent, and a powder. In addition, the oil phase part may further include one or more ingredients selected from the group consisting of a UV blocking agent, an emulsifier, and a solvent, and those skilled in the art may select and blend appropriate ingredients depending on a formulation or purpose of use of the cosmetic.

In one embodiment of the present disclosure, the organic UV blocking agent may be included in the water phase part or the oil phase part. The organic UV blocking agent ingredient included in the water phase part may be one or more selected from the group consisting of terephthalylidene dicamphor sulfonic acid, phenylbenzimidazole sulfonic acid, menthyl anthranilate, benzophenone-4, benzophenone-8, and disodium phenyldibenzimidazole tetrasulfonate. The organic UV blocking agent ingredient included in the oil phase part may include a material selected from the group consisting of octyl methoxycinnamate, ethylhexylsalicylate, homosalate, 4-methylbenzylidene camphor, drometrizole, drometrizole siloxane, digalloyl trioleate, diethylamino hydroxybenzoyl hexyl benzoate, diethylhexyl butamido triazone, methylene bis-benzotriazolyl tetramethylbutylphenol, benzophenone-3, butyl methoxydibenzoylmethane, bis-ethylhexyloxyphenol methoxyphenyl triazine, cinoxate, ethylhexyl dimethyl PABA, ethylhexyl methoxycinnamate, ethylhexyl triazone, octocrylene, isoamyl p-methoxycinnamate, polysilicone-15, and combinations thereof, and any organic compound harmless to the human body may be used in addition to the types.

In one embodiment of the present disclosure, the emulsifier may include fatty alcohols, glyceryl caprylate, polyglyceryl-3 laurate, polyglyceryl-3 distearate, glyceryl stearate, glyceryl stearate citrate, cetearyl olivate, sorbitan olivate, potassium cetyl phosphate, sodium or potassium stearate, sucrate, sucrose esters of fatty acids, lactate, and mixtures thereof, but is not limited thereto.

In one embodiment of the present disclosure, the pH adjusting agent may be used with one or more selected from the group consisting of triethanolamine, arginine, tromethamine, sodium hydroxide, and potassium hydroxide, preferably tromethamine, but is not limited thereto.

In one embodiment of the present disclosure, the emulsion stabilizer may consist of inulin lauryl carbamate, poloxamer 407, and mixtures thereof, but is not limited thereto.

The UV blocking cosmetic composition according to the present disclosure may be in the form of liquid, lotion, cream, aerosol, and solid or solid including powder, preferably a lotion composition. In the present disclosure, the formulation of the cosmetic composition may include solution, emulsion, gel, cream, suspension or paste formulations, but is not limited thereto. The cosmetic composition may be formulated into, for example, a makeup primer, a makeup base, a liquid or solid foundation, a concealer, a lipstick, a lip gloss, a powder, a lip liner, an eyeliner, a mascara, an eyebrow, an eye shadow, a blusher, a twin cake, a UV blocking agent, a lotion, a cream, an essence, or the like, but is not limited thereto.

In one embodiment of the present disclosure, the content of the organic UV blocking agent may be 0 part by weight to 30 parts by weight with respect to 100 parts by weight of the UV blocking agent composition, preferably 5 parts by weight to 20 parts by weight, and may be adjusted within an appropriate blending range according to a cosmetic blending limit set by each country.

In one embodiment of the present disclosure, in addition to the ingredients, the UV blocking agent composition may be appropriately blended with ingredients commonly blended in a cosmetic composition, such as oils, waxes, surfactants, pigments, cosmetic additives, powders, sugars, antioxidants, buffers, various extracts, stabilizers, preservatives, fragrances, etc., without departing from the effects of the present disclosure.

### [Modes for Invention]

Hereinafter, the present disclosure will be described in detail by Examples. However, the following Examples are just illustrative of the present disclosure, and the contents of the present disclosure are not limited to the following Examples.

### Example 1. Preparation of UV blocking cosmetic composition

A UV blocking cosmetic composition was prepared according to the ingredients and contents shown in Table 1 below.

**[Table 1]**

| **Classifica tion** | **Purpose of Use (Role)** | **Ingredient** | **Content (wt%)** | | | | |
|---|---|---|---|---|---|---|---|
| | | | **Ex. 1** | **Ex. 2** | **Ex. 3** | **Ex. 4** | **Ex. 5** |
| Water phase part | Solvent | WATER | 50.91 | 50.91 | 50.91 | 50.91 | 50.91 |
| | Skin conditioning agent | NIACINAMIDE | 2 | 2 | 2 | 2 | 2 |
| | | ADENOSINE | 0.04 | 0.04 | 0.04 | 0.04 | 0.04 |
| | | 1,3-PROPANDEDIOL | 3 | 3 | 3 | 3 | 3 |
| | | GLYCERIN | 2 | 2 | 2 | 2 | 2 |
| | | ETHYLHEXYLGL YCERIN | 0.2 | 0.2 | 0.2 | 0.2 | 0.2 |
| | | 1,2-HEXANEDIOL | 2 | 2 | 2 | 2 | 2 |
| | Emulsion stabilizer | INULIN LAURYL CARBAMATE | 0.8 | 0.8 | 0.8 | 0.8 | 0.8 |
| | | POLOXAMER 407 | 0.5 | 0.5 | 0.5 | 0.5 | 0.5 |
| | pH adjusting agent | TROMETHAMIN E | 0.4 | 0.4 | 0.4 | 0.4 | 0.4 |
| | UV blocking agent | (TDSA) TEREPHTHALYLI DENE DICAMPHOR SULFONIC ACID | 1.5 | 1.5 | 1.5 | 1.5 | 1.5 |
| | | **(TDST) TEREPHTHALY LIDENE DICAMPHOR SULFONIC ACID*TITANIU M DIOXIDE*** | **3** | **3** | **3** | **3** | **3** |
| | Thickener | XANTHAN GUM | 0.1 | 0.1 | 0.1 | 0.1 | 0.1 |
| | | **HYDROXYPROP YL METHYLCELLU LOSE STEAROXY ETHER** | **0.05** | **0.05** | | | |
| | | **Hydroxyethyl cellulose** | | | **0.05** | | |
| | | **Carboxymethyl cellulose** | | | | **0.05** | |
| | | **Hydroxyethyl methyl cellulose** | | | | | **0.05** |
| | Inorganic thickener | **HECTORITE** | **0.5** | | | | |
| | | **BENTONITE** | | **0.5** | | | |
| | Powder | SILICA | 1 | 1 | 1 | 1 | 1 |
| Oil phase part | UV blocking agent | ETHYLHEXYL TRIAZONE | 2 | 2 | 2 | 2 | 2 |
| | | BIS-ETHYLHEXYLO XYPHENOL METHOXYPHEN YL TRIAZINE | 1.5 | 1.5 | 1.5 | 1.5 | 1.5 |
| | | POLYSILICONE-15 | 1 | 1 | 1 | 1 | 1 |
| | | DROMETRIZOLE TRISILOXANE | 3 | 3 | 3 | 3 | 3 |
| | | DIETHYLAMINO HYDROXYBENZ OYL HEXYL BENZOATE | 2 | 2 | 2 | 2 | 2 |
| | Emulsifier | PALMITIC ACID*STEARIC ACID*MYRISTIC ACID | - | - | - | - | - |
| | | CETEARYL ALCOHOL | 0.5 | 0.5 | 0.5 | 0.5 | 0.5 |
| | | POLYGLYCERYL -3 DISTEARATE*GL YCERYL STEARATE CITRATE | 2.5 | 2.5 | 2.5 | 2.5 | 2.5 |
| | | BEHENYL ALCOHOL | 0.5 | 0.5 | 0.5 | 0.5 | 0.5 |
| | | GLYCERYL STEARATE | 1 | 1 | 1 | 1 | 1 |
| | Solvent | DIBUTYL ADIPATE | 6 | 6 | 6 | 6 | 6 |
| | | BUTYLOCTYL SALICYLATE | 6 | 6 | 6 | 6 | 6 |
| | | CAPRYLIC/CAPR IC/SUCCINIC TRIGLYCERIDE | 6 | 6 | 6 | 6 | 6 |
| | | TOTAL | 100 | 100 | 100 | 100 | 100 |

| | | | | | | | |
|---|---|---|---|---|---|---|---|
| (Unit: wt%) | | | | | | | |

The results of measuring the formulation formation, aggregation phenomenon, stability and in vitro SPF results of UV blocking cosmetic compositions prepared according to the ingredient contents of Examples 1 to 5 were confirmed as shown in Table 2 below. Specifically, it was confirmed that no aggregation phenomenon occurred in the compositions according to Examples 1 to 5, the stability of the formulation was excellent, and the SPF was 50 or higher, indicating an excellent UV blocking effect.

**[Table 2]**

| **Classification** | **Example 1** | **Example 2** | **Example 3** | **Example 4** | **Example 5** |
|---|---|---|---|---|---|
| Formulation formation | O | O | O | O | O |
| Aggregation phenomenon | X | X | X | X | X |
| Stability | O | O | O | O | O |
| *in vitro* SPF | 50 or higher | 50 or higher | 50 or higher | 50 or higher | 50 or higher |

### Experimental Example 1. Measurement of aggregation phenomenon, SPF (in vitro), etc. according to changes in composition

### 1-1. Test according to changes in content of organic-inorganic complex UV blocking ingredient (TDST)

For the corresponding test, compositions of Comparative Examples 1 to 5 were prepared. The compositions of Comparative Examples 1 to 5 were prepared in the same manner as Example 1 in the contents of other ingredients, except that there was a difference only in the content of the TDST ingredient, the content of the inorganic thickener, and the content of purified water according to the contents thereof, as shown in Table 3 below.

**[Table 3]**

| **Ingredient** | **Ex. 1** | **Com. Ex. 1** | **Com. Ex. 2** | **Com. Ex. 3** | **Com. Ex. 4** | **Com. Ex. 5** |
|---|---|---|---|---|---|---|
| WATER | 50.91 | 52.91 | 48.41 | 43.41 | 53.81 | 38.41 |
| (TDST) TEREPHTHALYLIDENE DICAMPHOR SULFONIC ACID*TITANIUM DIOXIDE* | 3 | 1 | 5 | 10 | 0.1 | 15 |
| HECTORITE | 0.5 | 0.5 | 1 | 1 | 0.5 | 1 |

| | | | | | | |
|---|---|---|---|---|---|---|
| (Unit: wt%) | | | | | | |

The results of confirming the formulation formation, aggregation phenomenon, stability and *in vitro* SPF results for the compositions prepared with the contents shown in Table 3 above were shown in Table 4 below.

**[Table 4]**

| **Classification** | **Com. Ex. 1** | **Com. Ex. 2** | **Com. Ex. 3** | **Com. Ex. 4** | **Com. Ex. 5** |
|---|---|---|---|---|---|
| Formulation formation | O | O | O | O | X |
| Aggregation phenomenon | X | X | X | X | O |
| Stability | O | O | O | O | - |
| *in vitro* SPF | 50 or higher | 50 or higher | 50 or higher | Less than 50 | 50 or higher |

As shown in Table 4 above, there was no problem when the organic-inorganic complex UV blocking material (TDST) was contained within 1 to 10 wt% based on the composition weight, but in Comparative Example 4 containing 0.1 wt% of TDST, the SPF was less than 50 to reduce a UV blocking effect, and in Comparative Example 5 containing 15 wt% of TDST, there was a problem in that the formulation was not properly formed.

### 1-2. Test according to changes in content of inorganic thickener

For the corresponding test, compositions of Comparative Examples 6 to 11 were prepared. The compositions of Comparative Examples 6 to 11 were prepared in the same manner as Example 1 in the contents of other ingredients, except that there was a difference only in the content of hectorite, and the content of purified water according to the content thereof, as shown in Table 5 below.

**[Table 5]**

| **Ingredient** | **Ex. 1** | **Com. Ex. 6** | **Com. Ex. 7** | **Com. Ex. 8** | **Com. Ex. 9** | **Com. Ex. 10** | **Com. Ex. 11** |
|---|---|---|---|---|---|---|---|
| WATER | 50.91 | 51.41 | 51.4 | 50.41 | 49.41 | 51.405 | 48.41 |
| HECTORITE | 0.5 | - | 0.01 | 1 | 2 | 0.005 | 3 |

| | | | | | | | |
|---|---|---|---|---|---|---|---|
| (Unit: wt%) | | | | | | | |

The results of confirming the formulation formation, aggregation phenomenon, and stability for the compositions prepared with the contents shown in Table 5 above were shown in Table 6 below.

**[Table 6]**

| **Classification** | **Com. Ex. 6** | **Com. Ex. 7** | **Com. Ex. 8** | **Com. Ex. 9** | **Com. Ex. 10** | **Com. Ex. 11** |
|---|---|---|---|---|---|---|
| Formulation formation | O | O | O | O | O | X |
| Aggregation phenomenon | X | X | X | X | O | X |
| Stability | X | O | O | O | X | - |

As shown in Table 6 above, in Comparative Example 6 without containing the hectorite inorganic thickener, the stability of the composition was reduced, and no problem occurred when the content ratio was within 0.01 to 2 wt%. However, in Comparative Example 10 containing 0.005 wt%, the stability of the composition was reduced, and in Comparative Example 11 containing 3 wt%, the formulation was not properly formed, and the spreadability became very heavy. Specifically, in the case of Comparative Examples 6 and 11, which had reduced stability, it was confirmed that an oil separation phenomenon occurred as the emulsification stability of the composition was decreased. In addition, when the same test was performed using bentonite as the inorganic thickener instead of hectorite (see Example 2), it was confirmed that there was no problem in formulation formation and composition stability, no aggregation phenomenon occurred, and spreadability was excellent, so that there was no difference from a case using hectorite.

### 1-3. Test according to changes in content of organic thickener (first thickener)

For the corresponding test, compositions of Comparative Examples 12 to 17 were prepared. The compositions of Comparative Examples 12 to 17 were prepared in the same manner as Example 1 in the contents of other ingredients, except that there was a difference only in the absence of hydroxypropyl methylcellulose stearoxy ether contained or the content thereof, and the content of purified water according to the content thereof, as shown in Table 7 below.

**[Table 7]**

| **Ingredient** | **Ex. 1** | **Com. Ex. 12** | **Com. Ex. 13** | **Com. Ex. 14** | **Com. Ex. 15** | **Com. Ex. 16** | **Com. Ex. 17** |
|---|---|---|---|---|---|---|---|
| WATER | 50.91 | 50.96 | 50.95 | 49.96 | 50.46 | 50.955 | 47.96 |
| HYDROXYPROPYL METHYLCELLULOS E STEAROXY ETHER | 0.05 | - | 0.01 | 1 | 0.5 | 0.005 | 3 |

| | | | | | | | |
|---|---|---|---|---|---|---|---|
| (Unit: wt%) | | | | | | | |

The results of confirming the formulation formation, aggregation phenomenon, and stability for the compositions prepared with the contents shown in Table 7 above were shown in Table 8 below.

**[Table 8]**

| **Classification** | **Com. Ex. 12** | **Com. Ex. 13** | **Com. Ex. 14** | **Com. Ex. 15** | **Com. Ex. 16** | **Com. Ex. 17** |
|---|---|---|---|---|---|---|
| Formulation formation | X | O | O | O | X | X |
| Aggregation phenomenon | O | X | X | X | O | X |
| Stability | X | O | O | O | - | - |
| Stickiness | - | None | None | Good | None | Stickiness |

As shown in Table 8 above, in Comparative Example 12 without containing hydroxypropyl methylcellulose stearoxy ether, the formulation was not properly formed, and an aggregation phenomenon occurred, but no problem occurred when the content ratio was within 0.01 to 1 wt%. However, in Comparative Example 16 containing 0.005 wt% of hydroxypropyl methylcellulose stearoxy ether, the formulation was not formed properly, and the aggregation phenomenon occurred, and in Comparative Example 17 containing 3 wt% of hydroxypropyl methylcellulose stearoxy ether, the formulation was not formed properly, and a problem of the formulation becoming stickier occurred. In addition, when the same test was performed by including 0.05 wt% of hydroxyethyl cellulose, carboxymethyl cellulose or hydroxyethyl methyl cellulose as an organic thickener instead of hydroxypropyl methylcellulose stearoxy ether (see Examples 3 to 5), it was confirmed that there was no problem in formulation formation or composition stability, and no aggregation phenomenon occurred.

### Experimental Example 2. Confirmation of SPF effect when applying TDST organic-inorganic complex

Comparative Example 18 was prepared by excluding a TDST ingredient from the ingredients shown in Example 1 and including 1.8 wt% of terephthalylidene dicamphor sulfonic acid and 1.8 wt% of titanium dioxide, and the compared results were shown in Table 9 below. The preparing content ratio of Comparative Example 18 was the same, except for the ingredients and the content of water according to the ingredients.

**[Table 9]**

| **Classification** | **Example 1** | **Comparative Example 18** |
|---|---|---|
| Formulation formation | O | O |
| Aggregation phenomenon | X | O |
| *in vitro* SPF | 73.4 | 32.3 |
| *in vitro* UVAPF | 16.78 (PA++++) | 15.4 |
| *in vivo* SPF | 50.1 (SPF50+) | - |
| *in vivo* PFA | PFA16.8 (PA++++) | - |

As can be seen in Table 9 above, in Comparative Example 18, the stability of the composition was reduced due to the occurrence of an aggregation phenomenon, and the SPF value of 32.3 was also significantly lower than that of Example 1, which was 73.4. It was shown that there was a better UV blocking effect when the organic-inorganic complex UV blocking ingredient (TDST) according to the present disclosure was contained in an appropriate amount and stably dispersed without an aggregation phenomenon within the formulation.

### Experimental Example 3. Confirmation of white turbidity of cosmetic composition

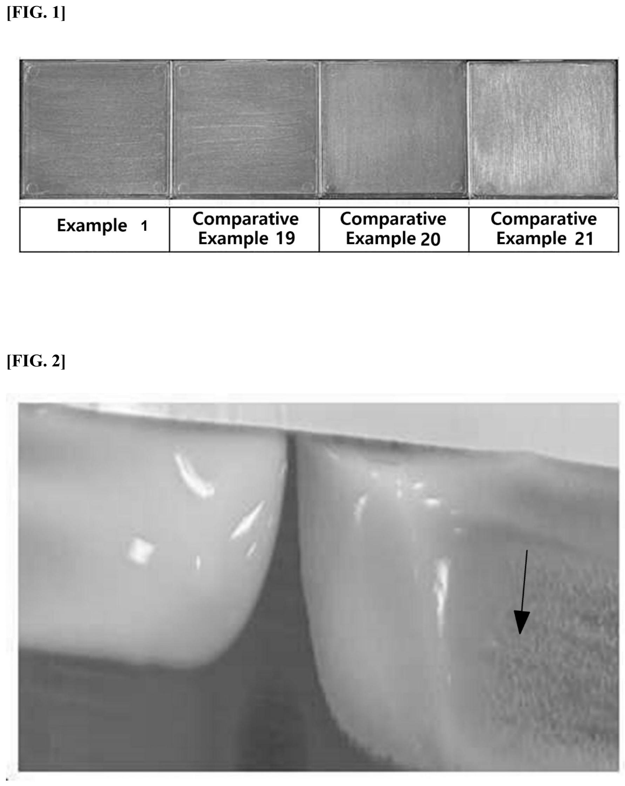

The white turbidity was confirmed according to the ingredients of a UV blocking agent shown in Table 10 below. The UV blocking agent was used with an organic UV blocking agent or an inorganic UV blocking agent (TiO₂) which was commonly sold on the market. Specifically, the results were shown in FIG. 1 by comparing the composition of Example 1 with compositions prepared using ingredients of an organic UV blocking agent (Comparative Example 19), an inorganic UV blocking agent and an organic UV blocking agent (Comparative Example 20), and an inorganic UV blocking agent (Comparative Example 21).

**[Table 10]**

| | **Example 1** | **Com. Ex. 19** | **Com. Ex. 20** | **Com. Ex. 21** |
|---|---|---|---|---|
| Organic-inorganic complex UV blocking agent (TDST) | O | - | - | |
| Inorganic UV blocking agent (TiO₂) | - | | O | O |
| Organic UV blocking agent | O | O | O | |
| Whitening phenomenon | Almost none | Almost none | Slightly present | Present |

As can be seen in FIG. 1, the whitening phenomenon, which was a problem that mainly occurred in inorganic UV blocking agents, was hardly observed in Example 1, and an emulsion with an excellent moisture content without stickiness could be formed, whereas a slight whitening phenomenon could be observed in Comparative Example 20, and a severe whitening phenomenon could be observed in Comparative Example 21.

### Experimental Example 4. Temporal stability test of cosmetic composition

A temporal stability test for the composition according to Example 1 was performed at 25°C for 6 months and 0°C and 40°C for 3 months, and the results were shown in Table 11 below by measuring the hardness and pH at the next day, 1 month, 3 months, and 6 months, respectively.

**[Table 11]**

| Example | Next day | 1 month | 3 months | 6 months |
|---|---|---|---|---|
| 5°C | - | No problem | No problem | - |
| 25°C | No problem | No problem | No problem | No problem |
| 40°C | - | No problem | No problem | - |

As can be confirmed in Table 11 above, as results of performing the temporal stability test, no oil separation or re-aggregation phenomenon was observed, and it was confirmed that the formulation was maintained evenly for 6 months or more. In comparison, in Comparative Example 22 containing 0.1 wt% of polyacrylate crosspolymer-6, excluding the hydroxypropyl methylcellulose stearoxy ether thickener ingredient in Example 1, the results of observing the composition after 3 months were shown in FIG. 2.

As shown in FIG. 2, in the case of the left side (Example 1), the oil separation and aggregation phenomenon did not occur at all, but in the case of the right side (Comparative Example 22), it was confirmed that the surface gloss was reduced and the aggregation phenomenon occurred (see arrow mark).

## Claims

1. A UV blocking cosmetic composition comprising:
a UV blocking material consisting of an organic-inorganic complex, an organic thickener, and an inorganic thickener,
wherein the UV blocking material consisting of the organic-inorganic complex is formed from terephthalylidene dicamphor sulfonic acid and titanium dioxide.

2. The UV blocking cosmetic composition of claim 1, wherein the UV blocking material consisting of the organic-inorganic complex is contained in an amount of 1 to 10 wt% based on a total weight of the composition.

3. The UV blocking cosmetic composition of claim 1, wherein the organic thickener includes a first thickener and a second thickener.

4. The UV blocking cosmetic composition of claim 3, wherein the first thickener is one or more selected from the group consisting of hydroxypropyl methylcellulose stearoxy ether, hydroxyethyl cellulose, carboxymethyl cellulose, and hydroxyethyl methyl cellulose.

5. The UV blocking cosmetic composition of claim 3, wherein the second thickener is one or more selected from the group consisting of cellulose gum, xanthan gum, gellan gum, guar gum, dehydroxanthan gum, carob bean gum, tamarind seed gum, mastic gum, carob gum, diutan gum, and tara gum.

6. The UV blocking cosmetic composition of claim 3, wherein the first thickener is contained in an amount of 0.01 to 1 wt% based on a total weight of the composition.

7. The UV blocking cosmetic composition of claim 1, wherein the inorganic thickener is hectorite or bentonite.

8. The UV blocking cosmetic composition of claim 1, wherein the organic thickener is contained in an amount of 0.01 to 2 wt% based on a total weight of the composition.

9. The UV blocking cosmetic composition of claim 1, further comprising:
an emulsion stabilizer and a pH adjusting agent,
wherein the emulsion stabilizer is contained in an amount of 0.1 to 5 wt% based on a total weight of the composition and the pH adjusting agent is contained in an amount of 0.1 to 2 wt% based on the total weight of the composition.

10. The UV blocking cosmetic composition of claim 9, wherein the emulsion stabilizer consists of inulin lauryl carbamate, poloxamer 407, or mixtures thereof.

11. The UV blocking cosmetic composition of claim 9, wherein the pH adjusting agent is one or more selected from the group consisting of triethanolamine, arginine, tromethamine, sodium hydroxide, and potassium hydroxide.
